Europäisches Patentamt

European Patent Office

Office européen des brevets

(11) Publication number: **0 289 165**

**A1**

# (12) EUROPEAN PATENT APPLICATION

(21) Application number: 88303264.1

(22) Date of filing: **12.04.88**

(51) Int. Cl.⁴: **G01F 23/00 , A61M 5/00**

(30) Priority: **16.04.87 GB 8709191**

(43) Date of publication of application:
**02.11.88 Bulletin 88/44**

(84) Designated Contracting States:
**AT BE CH DE ES FR GB GR IT LI LU NL SE**

(71) Applicant: **Goble, Nigel Mark**
**Meadow View Station Road Pilning**
**Bristol Avon(GB)**

Applicant: **Goble, Colin Charles Owen**
**Flat 4 Bakers Court Clive Road**
**Canton Cardiff Glamorgan(GB)**

(72) Inventor: **Goble, Nigel Mark**
**Meadow View Station Road Pilning**
**Bristol Avon(GB)**
Inventor: **Goble, Colin Charles Owen**
**Flat 4 Bakers Court Clive Road**
**Canton Cardiff Glamorgan(GB)**

(74) Representative: **Harrison, Ivor Stanley et al**
**Withers & Rogers 4 Dyer's Building Holborn**
**London EC1N 2JT(GB)**

(54) **Liquid quantity indicator.**

(57) Apparatus for sensing the volume of liquid remaining in a flexible container from which liquid is withdrawn, such as an irrigation or infusion bag to provide an alarm signal when the volume falls below a predetermined level comprises sensing jaw members (41, 42) biassed towards the closed position and means (50, 51) operatively connected to at least one of said jaw members (41, 42) for emitting a signal in dependence upon the spacing of the jaw members (41, 42), the jaw members (41, 42) being positionable against the walls of the container whereby the members move towards the closed position as the volume of liquid falls. It is preferred that the apparatus is suspended from the lower edge of the container.

Preferably the sensing jaw members (41, 42) are shaped to define inwardly-directed front end regions and spaced-apart central regions whereby, in use, the front end regions are positionable in contact with the walls of the container and the spaced-apart regions accommodate without contact the lowermost portion of the container holding residual liquid after the level has fallen below the front end regions.

FIG 4

# LIQUID QUANTITY INDICATOR

This invention relates to a liquid quantity indicator and in particular provides means for indicating when the quantity of a consumable liquid in a flexible-walled container falls below a predetermined amount.

In medicine and surgery, it is frequently the case that liquids for introduction into the body are contained in a flexible-walled bag suspended from a supporting structure, the liquid being gravity-fed from the bag to the body via a flexible catheter. Such techniques are used for example for intravenous infusion of saline and other solutions and for irrigation of certain organs such as the prostate gland during surgical operations.

Irrespective of particular circumstances relating to the use of the technique, it is desirable to provide an automatic indication of when the bag is almost empty, in order that a filled bag can be made ready for substitution for the nearly empty one. This is of particular importance during irrigation, when the flow of liquid is relatively high and consequently a full bag becomes empty in a fairly short time. In order to provide such an indication, drip counters or other devices for measuring and integrating the flowrate have been employed, the device emitting a warning signal when the initial volume of liquid is calculated to have been almost consumed. As an alternative, it has been proposed to employ a device which gives a warning signal when the weight of the bag and contents falls below a predetermined value. Both such prior proposals, however, are subject to external influences or inaccuracies in that the triggering of the warning signal is not necessarily related to the absolute quantity of liquid in the bag but is rather related to a derived value or parameter.

It is an object of the present invention to provide a liquid quantity indicator of the type as hereinbefore described which is straightforward and reliable in operation and which emits a signal which is directly related to the absolute quantity of liquid remaining in the container.

According to the invention, apparatus for sensing the volume of liquid remaining in a flexible container from which liquid is withdrawn to provide a signal when the volume falls below a predetermined level comprises sensing jaw members biassed towards the closed position and means operatively connected to at least one of said jaw members for emitting a signal in dependence upon the spacing of the jaw members, the jaw members being positionable against the walls of the container whereby the said members move towards the closed position as the volume of liquid falls.

The invention is of particular use for determining when the quantity of a consumable liquid in a flexible-walled bag falls below a predetermined amount, for example in medical and surgical applications, the spacing of the jaw members being a function of the liquid remaining in the bag.

The critical spacing of the sensing jaws at which the signal emission is generated may be either the angular spacing between them or the linear distance between selected points or zones thereon. Furthermore, either a single signal may be set for emission at a predetermined critical spacing or a multiple or graduated signal may be set for emission initially at a threshold spacing and thereafter at one or more predetermined critical spacing values.

The sensing jaw members of a device according to the invention are positioned in use one on either side of the lower or discharge end portion of a flexible-walled container and are maintained in spaced-apart relationship against the bias by the outward pressure exerted on the walls by the liquid in the container. Absence of liquid in the container, or at least insufficient liquid to maintain the jaws spaced-apart above the threshold or critical spacing value, results in the emission of a signal, which may be an audible or a visual signal or an input to a data storage system, for example, or may be an infra-red signal transmitted to a ward nursing station together with an identification of the patient.

Devices according to the invention are particularly suitable for use with gravity-feed containers and accordingly it is preferred that the jaw members are positionable at the lower end of the container, preferably so that the lower edge of the container is located in the throat of the jaws. Suspension means may be provided for maintaining the device in the desired position with respect to the container, such suspension means comprising for example a hook mounted between the jaw members in the throat thereof for engagement with a suitable eye or corresponding hook formed in or attached to the lower edge region of the container, or separate biassed clamping jaw members which clamp on the lower seam of the container or around the outlet tube thereof.

The rear portions of the sensing jaw members may be maintained in spaced-apart relationship, for example by being connected to a bridging member, at least one of the jaw members, but preferably both, being pivotably connected to the bridging member. In such an arrangement, the bias, which acts on at least one hinged jaw member, tends to close the jaw members together so that, in the absence of a container or other restraint, they meet at the front or tip portions thereof. Alternatively and/or additionally, the jaw members are shaped, for example by being cranked, to define inwardly-directed front end regions and spaced-apart central regions whereby, in use, the front end regions of the jaw members are positionable in contact with the walls and the spaced-apart regions accommodate without contact the lowermost portion of the container holding residual liquid after the level has fallen below the front end regions.

It is to be understood that the critical spacing of the jaw members at which the signal is emitted may be set to be either when the surface of liquid in the container is below the level at which the jaw members contact the walls of the container or, alternatively, while the surface is still above the level of the jaw members, the jaw members having nevertheless moved towards one another owing to the reduced pressure exerted on the walls of the container by the a reduced volume of liquid compared with the full condition, the set level being a function of the overall capacity of the bag, the rate of flow of liquid being withdrawn, and the period of notice required before the bag is empty.

According to a preferred arrangement, the sensing jaw members are pivotably connected together at their rear or root ends and are biassed towards the closed position, and opening pressure may be exerted against the bias by a pair of clamping jaws co-axially mounted with and between the sensing jaws, the clamping jaws being also and independently biassed towards the closed position, whereby opening the clamping jaws for attachment to a flexible-walled container or to the outlet tube thereof causes the sensing jaws also to open to be positioned on either side of the container. The sensing jaws, in such an arrangement, are outwardly bowed or otherwise shaped to define a space therebetween when the tips or front end regions are proximate, the space being for accommodation of the lowermost portion of the container when it is in the condition of being almost empty. The sensing jaws may carry a switch means, for example a microswitch, to activate the signal-emitting circuitry when the jaws are in this position, the microswitch optionally being adjustably mounted on the jaws for adjustment of the threshold spacing of the jaws for activation of the circuitry. The clamping jaws may be attached to handles for facilitating the opening thereof.

Preferably the sensing jaw members are arranged so that they can be angularly offset to either side to accommodate containers from which the exit tube does not emerge in alignment with the longitudinal axial plane of the container. Conveniently, the jaw members can swing by an amount up to plus or minus 5° about the axis when they are held open by the clamping jaws, and more when the sensing jaws are not held open by the clamping jaws.

The signal-emitting means generally comprises a microswitch connected to one or both of the sensing jaw members and a piezoelectric alarm, controlled by a microchip and powered by an electrochemical cell. However, other switching devices could be used, such as a light sensor on one jaw member which is positioned to receive a beam of light from the other jaw member when the jaw members are at a predetermined angular spacing. As a further alternative, an electrical contact, which is preferably adjustable, on one or each jaw member may be arranged to touch a fixed contact mounted between the jaw members at a predetermined position of the jaw member with respect to the fixed contact, thereby closing a signal-generating circuit. Optionally, a cell-condition circuit is included to check the voltage of the power source on attachment of the device to a container.

Optionally, to avoid inadvertent power drain, a restraint such as a mechanical clip or catch may be included to hold the sensing jaws apart against the biassing pressure to an extent at least sufficient to prevent the switch means from closing when the apparatus is not is use.

The invention also includes a method for sensing the volume of liquid remaining in a flexible container from which liquid is withdrawn to provide a signal when the volume falls below a predetermined level, the method comprising suspending apparatus according to the invention from the lower end of the container so that the sensing jaw members are positioned against the walls of the container and are held spaced apart by the pressure of liquid therein acting through the walls thereof and allowing liquid to leave the container through an exit tube in the lower portion of the container, whereby the volume of liquid therein falls and allows the jaws to close together to cause a signal to be emitted as the jaws attain a predetermined spacing with respect to each other, the said spacing being a function of the volume of liquid remaining the container.

Embodiments of the invention will now be described by way of exmaple with reference to the accompanying drawings, in which:

Figure 1 is an end elevation of a device according to one aspect of the invention with the jaws in the open position;

Figure 2 is a diagram illustrating the device of Figure 1 in use with a filled irrigation bag;

Figure 3 is a diagram illustrating the device as in Figure 2 but with the irrigation bag almost empty;

Figure 4 is a diagram of a further device according to the invention;

Figures 5 and 6 illustrate yet a further device according to the invention with the jaws open and closed respectively and

Figure 7 is a circuit diagram suitable for use in apparatus according to the invention.

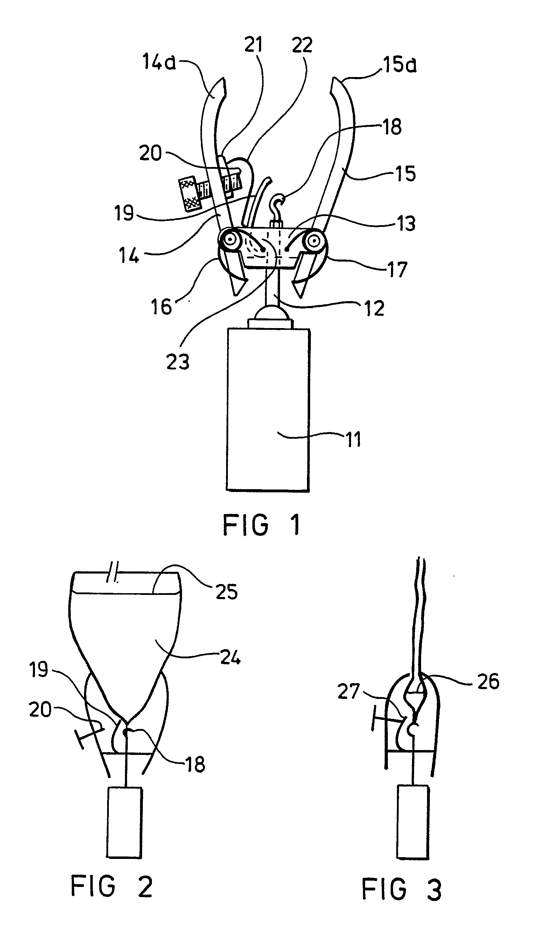

Referring firstly to Figure 1, a housing for a piezoelectric signal-emitting circuit and power source is shown at 11 and carries an upstanding hollow cylinder 12 to which is attached a bridge 13. To opposite ends of the bridge are pivoted jaw members 14, 15 having inwardly-directed sloping end regions 14a, 15a which are biassed together by respective springs 16, 17. The cylinder also carries a hook 18 for attachment to the lower edge of an irrigation or other bag (not shown). A fixed contact 20 is attached to the bridge and an adjustable contact 20 is attached to jaw member 14 via a contact block 21. A flexible wire 22 connects the contact block with the circuit via the inside of the cylinder 12; a further flexible wire 23 connects the fixed contact 19 to the circuit.

Referring to Figure 2, the hook 18 is attached to the lower edge of an irrigation bag 24 containing a liquid the surface of which is shown at 25; the outward pressure exerted by the liquid in the bag maintains the jaw members in the open position against the spring pressure so that the movable contact 20 is held away from the fixed contact 19.

Referring to Figure 3, the surface of the liquid 26 has dropped with the result that the jaw members have closed under pressure of the springs and the contacts have closed the circuit at 27. It is to be noted that the arrangement of the bridge 13 in combination with the inward-sloping ends of the jaw members allows the jaw members to close to their fullest extent despite some liquid remaining in the bag, thus giving a warning signal before the bag becomes completely empty. It is, however, to be understood that the warning signal could be activated at an earlier stage, before the level of the liquid surface had dropped below the inward-sloping ends of the jaw members but nevertheless as the said ends had closed together to a predeterminable extent.

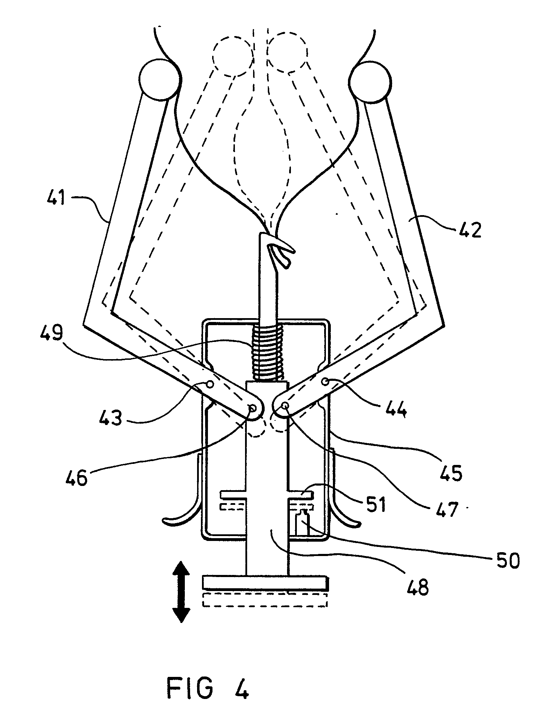

Referring to Figure 4, a pair of cranked jaw members are shown at 41, 42, each jaw member being pivotably connected at 43 and 44 respectively to the sides of a housing 45, the rear end regions of the jaw members being thereby maintained in spaced-apart relationship. The inner rear ends of the jaw members are pivotably connected at 46 and 47 respectively to opposed sides of a rod member 48 which can reciprocate longitudinally in the housing and is biassed downwardly (that is, to close the jaw members) by a compression spring 49. A microswitch 50 is mounted within the housing for activation by a lug 51 formed on the rod member. The position of the jaw members and rod member with a filled bag is shown in solid outline and with an almost empty bag in dashed outline.

With an arrangement as illustrated in Figure 4 with the jaw members linked together and the microswitch being activated by the linking member, in the particular embodiment illustrated by means of the rod member 48, the jaw members can only pivot in concert and in dependence on each other and the microswitch cannot be prematurely activated for example by one jaw member sticking and the inward movement being taken up in total by the other jaw member.

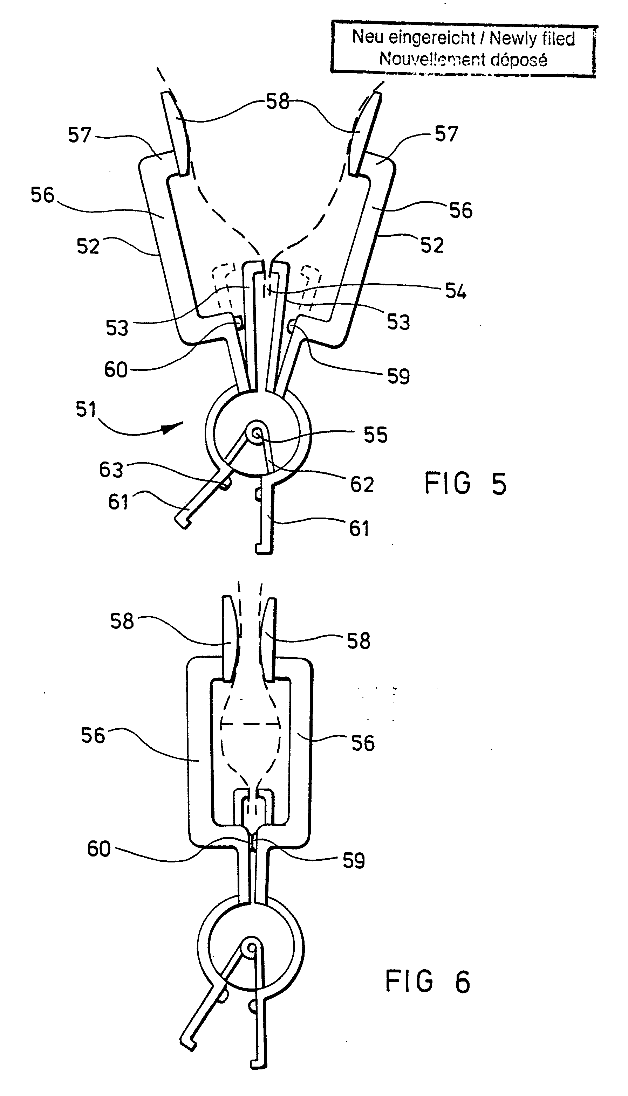

Figures 5 and 6 illustrate yet a further embodiment of the invention, shown attached to the lower portion of an irrigation bag with the bag full (Figure 5) and almost empty (Figure 6). Referring to Figure 5, the device shown generally at 51 includes a pair of sensing jaws 52 and a pair of suspension jaws 53 for gripping around the exit tube 54 of the irrigation bag. The sensing jaws and the suspension jaws are independently biassed towards the closed position and are co-axially mounted around pivot 55. The sensing jaws are shaped so that a central region 56 of each jaw is spaced away from the corresponding region of the other jaw to define a space therebetween, the head regions 57 being shaped so that they extend towards each other and terminating in bag-engaging portions 58. The lower or root end region of one jaw carries a microswitch 59 and the corresponding region of the other jaw carries a pad 60 or other contact for actuating the microswitch on the closing together of the jaws; the switch and/or the contact may be adjustably mounted on the jaw to enable the sensitivity of the device to be adjusted.

The suspension jaws carry a pair of handles 61 which on being squeezed together between thumb and finger open the jaws against the biassing pressure exerted by spring 62. On opening, the suspension jaws cause the sensing jaws to open by direct pressure thereon, against the biassing pressure exerted by a further spring similar to spring 62 but not shown in the drawings. The handles 61 carry a further microswitch 63 which activates a cell-test circuit to test the voltage of the power cell. The cell-test circuit is arranged to give a positive indication of the condition of the power cell provided that there is sufficient power to provide at least 24 hours' further continuous operation.

In use and as shown in Figure 5, the suspension jaws 53 are opened by squeezing together the handles 61; the suspension jaws, at the position shown by the dashed outline, then hold open the sensing jaws 52 to an extent to permit them to be positioned on either side of the walls of the bag. The handles are then released, whereupon the suspension jaws close around the exit tube from the bag and the sensing jaws are maintained in the open position by the pressure exerted by the liquid in the bag. As the bag empties, however, and as shown in Figure 6, the sensing jaws are permitted to close together to a proximate position at or by which the microswitch contacts close, while some liquid remains in the bag and accommodated in the space between the central regions 56 of the sensing jaws without any outward pressure being exerted thereon.

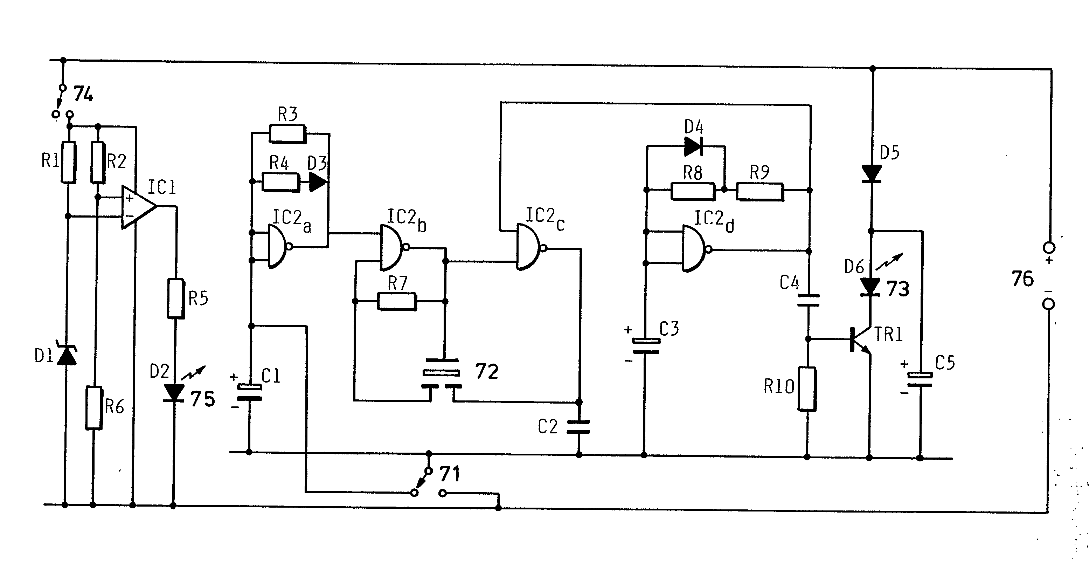

Figure 7 illustrates an electrical circuit including a power cell test facility, in which the sensing jaws switch is indicated 71, an audible alarm is indicated 72, a visual alarm is indicated 73, a power test switch is indicated 74, and an indicator light for signifying that the power cell is in good condition is indicated 75. The circuit is powered by a 6 volt cell 76. On the closing of the switch 71, the audible alarm is activated for only a limited period, typically 10-20 seconds, whereas the visual alarm flashes indefinitely until manually cancelled. Other components in the diagram include one or more resistors (R), diodes (D), capacitors (C), operational amplifiers (IC), quad Schmidt NAND gates ($IC^2$) and transistors (TR).

## Claims

1. Apparatus for sensing the volume of liquid remaining in a flexible container from which liquid is withdrawn to provide a signal when the volume falls below a predetermined level, characterised in that the apparatus comprises sensing jaw members biassed towards the closed position and means operatively connected to at least one of said jaw members for emitting a signal in dependence upon the spacing of the jaw members, the jaw members being positionable against the walls of the container whereby the said members move towards the closed position as the volume of liquid falls.

2. Apparatus according to claim 1, characterised in that the apparatus includes means for suspending it at the lower end of the container, the lower edge of the container being located between the jaws.

3. Apparatus according to claim 2, characterised in that the suspension means comprise clamping jaw members for clamping at the lower seam of the container or around the outlet tube therefrom.

4. Apparatus according to any of claims 1 to 3, characterised in that the sensing jaw members are shaped to define inwardly-directed front end regions and spaced-apart central regions whereby, in use, the front end regions are positionable in contact with the walls of the container and the spaced-apart regions accommodate without contact the lowermost portion of the container holding residual liquid after the level has fallen below the front end regions.

5. Apparatus according to any preceding claim, characterised in that the sensing jaw members are pivotally connected together at their rear ends.

6. Apparatus according to claim 5 as dependent on claim 3, characterised in that the clamping jaw members are coaxially mounted with and between the sensing jaw members, the clamping jaws being independently biassed towards the closed position.

7. Apparatus according to any preceding claim, characterised in that the signal-emitting means comprises a switch connected to one or both of the sensing jaw members and an alarm controlled by a microchip and powered by an electrochemical cell.

8. Apparatus according to claim 7, characterised in that the apparatus further includes a cell-condition test circuit operative on opening the jaws to attach the device to a container.

9. A method for sensing the volume of liquid remaining in a flexible container from which liquid is withdrawn to provide a signal when the volume falls below a predetermined level, characterised in that the method comprises suspending apparatus according to any of claims 1 to 8 from the lower end of the container so that the sensing jaw members are positioned against the walls of the container and are held spaced apart by the pressure of liquid therein acting through the walls thereof and allowing liquid to leave the container through an exit tube in the lower portion of the container, whereby the volume of liquid therein falls and allows the jaws to close together to cause a signal to be emitted as the jaws attain a predetermined spacing with respect to each other, the said spacing being a function of the volume of liquid remaining in the container.

FIG 1

FIG 2

FIG 3

FIG 4

FIG 5

FIG 6

0 289 165

| | DOCUMENTS CONSIDERED TO BE RELEVANT | | | EP 88303264.1 |
|---|---|---|---|---|
| **Category** | Citation of document with indication, where appropriate, of relevant passages | | Relevant to claim | CLASSIFICATION OF THE APPLICATION (Int. Cl.4) |
| X | US - A - 4 378 014 (ELKOW) <br> * Abstract; fig. 1-9 * | | 1,2,3, 5,7,9 | G 01 F 23/00 <br> A 61 M 5/00 |
| A | | | 4,6 | |
| A | EP - A1 - 0 210 142 (KABI VITRUM) <br> * Abstract; fig. 1-5 * | | 1,9 | |
| A | US - A - 3 777 697 (WOESSNER) <br> * Abstract; fig. 1-5 * | | 1,9 | |
| A | GB - A - 1 417 111 (TRIADYNAMICS) <br> * Claim 1; fig. 1-5 * <br> ---- | | 1,9 | |
| | | | | **TECHNICAL FIELDS SEARCHED (Int. Cl.4)** <br> G 01 F <br> A 61 M |

The present search report has been drawn up for all claims

| Place of search | Date of completion of the search | Examiner |
|---|---|---|
| VIENNA | 25-07-1988 | GRONAU |